(19) Europäisches Patentamt
European Patent Office
Office européen des brevets

(11) Publication number: 0 249 401 A1

(12) EUROPEAN PATENT APPLICATION

(21) Application number: 87304962.1

(22) Date of filing: 04.06.87

(51) Int. Cl.[3]: C 07 K 5/08
A 61 K 31/195

(30) Priority: 05.06.86 JP 131313/86

(43) Date of publication of application: 16.12.87 Bulletin 87/51

(84) Designated Contracting States: AT BE CH DE FR GB IT LI NL SE

(71) Applicant: YAMANOUCHI PHARMACEUTICAL CO. LTD.
No. 3-11 Nihonbashi-Honcho, 2-chome Chuo-ku
Tokyo(JP)

(72) Inventor: Nishikori, Koji
1-35, Matsubara 4-chome
Konosu-shi Saitama(JP)

(72) Inventor: Nagano, Yoshinobu
15-14, Midori-cho 2-chome
Tanashi-shi Tokyo(JP)

(72) Inventor: Kato, Miyuki
366-1, Shukurenji
Kashiwa-shi Chiba(JP)

(74) Representative: Geering, Keith Edwin et al,
REDDIE & GROSE 16 Theobalds Road
London WC1X 8PL(GB)

(54) **Antianemic agent.**

(57) An antianemic agent comprising as an effective component a glutathione monoalkyl ester represented by general formula :

```
H2NCHCH2CH2CONHCHCONHCH2COOR
    |               |
    COOH            CH2SH
```

wherein R represents an alkyl group, or a salt thereof.

The drugs of the present invention are useful as preventive and therapeutic agents for anemic conditions, particularly human hemolytic anemia.

EP 0 249 401 A1

0249401

# ANTIANEMIC AGENT

The present invention relates to antianemic agents.

The drugs of the present invention are useful as preventive and therapeutic agents for conditions caused by hemolysis, particularly human hemolytic anemia.

Anemia is a state in which the red blood cell count and the concentration of hemoglobin decrease below normal levels and there are known various morbid states such as blood loss anemia, bothriocephalus anemia, pernicious anemia, hemolytic anemia, aplastic anemia, etc.

Hemolytic anemia is a condition caused by hemolysis - erythroclasis in which erythrocyte membranes are destroyed and hemoglobin flows out of red blood cells.

Clinical symptons associated with hemolytic anemia include conditions based on accentuation in erythroclasis such as anemia, indirect increase in serum bilirubin, short life span of erythrocytes, reduction in serum haptoglobin, increase of urobin in feces and urine, splenomegaly, etc.; conditions based on increased erythrocyte productivity such as increase in net erythrocytes, erythrocythemia, myeloblast hyperplasia, change of bones on X rays, etc.

For prevention and therapy of these conditions, there have been hitherto adopted isolation of spleen, steroid hormone therapy, blood transfusion, administration of hematopoietic agents, administration

0249401

of vitamins, etc. However, these treatments have the problems that their effects are not satisfactory, hemolysis is induced by transfusion of plasma, etc., and satisfactory treatment has not been established heretofore.

The present inventors have made extensive investigations with a view to stabilizing red blood cell membranes and preventing erythroclasis to enhance the function of red blood cells and in turn prevent hemolytic anemia and improve various morbid states based on reduced function of red blood cells. As a result, it has been found that glutathione monoalkyl esters have marked effects for the prevention and therapy of hemolytic anemia. The glutathione monoalkyl esters are esters of the glycine carboxyl group of glutathione and some are reported, when de-esterified to the free carboxyl form, to detoxicate drugs and protect cells from oxygen and its metabolites (for example, peroxides), free radicals, externally given compounds, etc.

The present invention provides an antianemic agent comprising as an effective component at least one compound selected from glutathione monoalkyl esters of general formula (I):

$$\begin{array}{l} H_2N\underset{\displaystyle COOH}{\underset{|}{C}}HCH_2CH_2CONH\underset{\displaystyle CH_2SH}{\underset{|}{C}}HCONHCH_2COOR \end{array} \qquad (I)$$

wherein R represents an alkyl group, and salts thereof.

In formula (I) the alkyl group R is preferably a straight or branched carbon chain having 1 to 10 carbon atoms.

Accordingly, specific examples include methyl, ethyl, propyl, isopropyl, butyl, isobutyl, tert-butyl, pentyl, isopentyl, tert-pentyl, 1-methylbutyl, hexyl, isohexyl, 2-methylpentyl, 1-ethylbutyl, heptyl, octyl, and nonyl groups etc.

Formula I compounds and their salts may be made as follows :

(II)

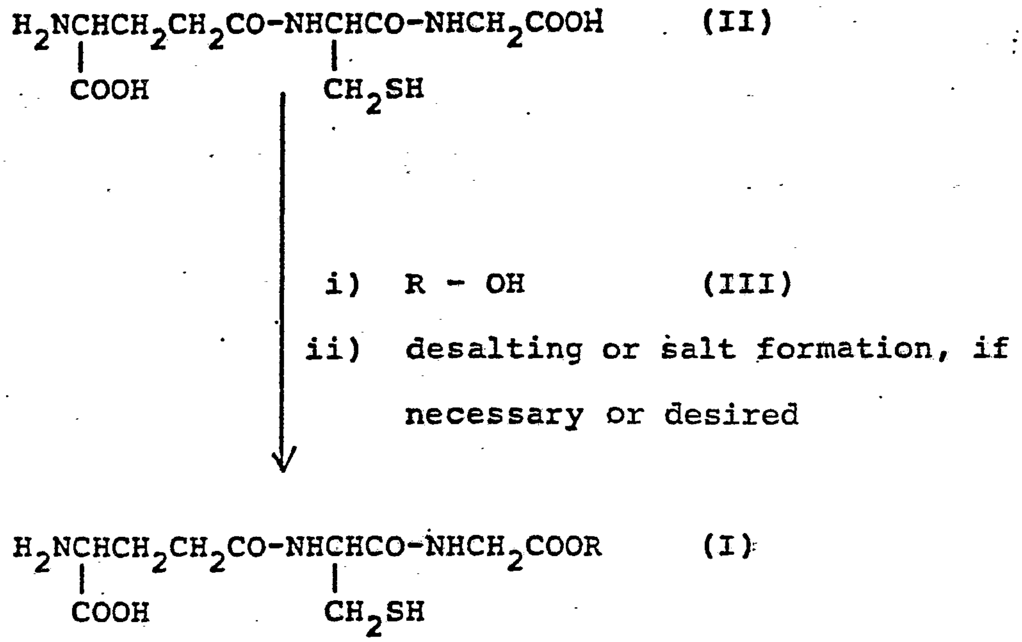


(I)

wherein R is an alkyl group.

This process can be carried out by reacting glutathione (II) with alcohol (III) in the presence of acid (for example, sulfuric acid) to give salt(s) of glutathione monoalkyl ester and then subjecting the salt(s) to desalting or salt formation, if necessary or desired.

Compounds (I) are preferably administered in free form; they may also be administered in the form of salts with inorganic acids (such as hydrochlorides, nitrates, sulfates, etc.) or with organic acids (such as citrates, p-toluenesulfonates, maleates, etc). When compounds are given in salt form they may be subjected to a desalting treatment, or administered with addition of bases such as sodium hydrogen carbonate that do not adversely affect the living body.

The compounds (I) and their salts can be formed into tablets,

powders, granulates, granules, capsules, pills, liquids, injections, etc., using conventional carriers, excipients and other additives for medical preparations which are administered orally or parenterally. The dose may be controlled according to administration route, body weight, age, condition of patient, etc. but a daily dose of several tens to 100 mg is generally suitable for an adult.

Compounds (I) and their salts show acute toxicity ($LD_{50}$) on intraperitoneal injection into mice, e.g. approximately 5.3 g/kg in the case of the isopropyl ester.

The antianemic agent of the present invention can be effective, by stabilizing red blood cell membranes, for treatment of and improvement in hereditary hemolytic anemia, erythrocyte abnormality, erythrocyte enzyme abnormality, autoimmune hemolytic anemia, porphyrinemia, physico-chemical hemolytic anemia and reduction in various other functions of erythrocytes.

The present invention is illustrated by the examples below. Processes for preparing glutathione monoisopropyl ester and monoethyl ester are given as reference examples.

Reference Example 1 Preparation of glutathione monoisopropyl ester

(1) To 800 ml of isopropyl alcohol is added 124 g of glutathione and 42 ml of 95% sulfuric acid is added dropwise to

the mixture while stirring. Heat evolves but there is no necessity to cool the mixture. Approximately an hour later, the mixture becomes a homogeneous solution and almost 24 hours after, crystals of glutathione monoisopropyl ester (isopropyl γ-L-glutamyl-L-cysteinylglycinate) sulfate begin to crystalize but stirring is further continued overnight. The crystals are taken by filtration, washed with 200 ml of isopropyl alcohol and dried under reduced pressure to give 88.5 g of the sulfate.

A part of the sulfate is recrystallized from a water-isopropyl alcohol mixture (mixing ratio: 1:5) to purify it, and is made a sample for analysis.

Melting point: 145 - 150°C

Elemental analysis (as $C_{13}H_{23}N_3O_6S.1/2H_2SO_4.1/2H_2O$)

| | C | H | N | S |
|---|---|---|---|---|
| Calcd. (%) | 38.32 | 6.18 | 10.31 | 11.80 |
| Found (%) | 38.11 | 6.34 | 10.32 | 12.10 |

(2) In 1.2 liters of water is dissolved 50.0 g of the unpurified sulfate obtained in (1) and the solution is charged into 1.5 liters of HP-20 , which is eluted with water and then with a methanol-water mixture (mixing ratio: 1:1) to give 2.5 liters of fractions containing the desired product. After concentrating the fractions, the concentrate is freeze dried to give 33.8 g of glutathione monoisopropyl ester.

(i) Melting point: 184 - 186°C

(ii) Infrared absorption spectrum (KBr, $cm^{-1}$

1730, 1635, 1525, 1400, 1370, 1205, 1100

(iii) Specific rotary power ($[\alpha]_D^{21}$)

-31.0 (c = 1.0, water)

(vi) Nuclear magnetic resonance spectrum (DMSO-$d_6$, δppm)

1.20 (6H, d, J=6Hz)

1.72 - 2.16 (2H, m)

2.20 - 2.40 (2H, m)

2.64 - 2.86 (2H, m)

3.20 - 3.56 (1H, m)

3.80 (2H, s)

4.20 - 4.60 (1H, m)

4.68 - 5.08 (1H, m)

Reference Example 2 Preparation of glutathione monoethyl ester

Glutathione monoethyl ester (ethyl γ-L-glutamyl-L-cysteinyl glycinate) is obtained in a manner similar to Reference Example 1.

(i) Infrared absorption spectrum (KBr, $cm^{-1}$

1735, 1635, 1520, 1400, 1200

(ii) Specific rotary power ($[\alpha]_D^{21}$)

-27.2 (c = 1.0, water)

(iii) Nuclear magnetic resonance spectrum (DMSO-$d_6$, δppm)

1.16 (3H, t, J=7)

1.70 - 2.04 (2H, m)

2.16 - 2.40 (2H, m)

2.60 - 2.82 (2H, m)

3.16 - 3.40 (1H, m)

3.80 (2H, s)

4.04 (2H, q, J=7)

4.20 - 4.48 (1H, m)

Example 1

Composition for Injection

Glutathione monoisopropyl ester sulfate is suspended in purified water in a concentration of 45 mg/ml and the suspension is cooled to below 5°C. Approximately 2-fold mols of sodium hydrogen carbonate is added to dissolve the ester sulfate. The solution pH is adjusted to 4, and then the concentration to 40 mg/ml, followed by sterile filtration; 25 ml aliquots of the solution are charged into vials and freeze dried.

Example 2

Hemolysis prevention

Method

Male Sprague Dawley rats (7 to 8 weeks old) are anesthetized with ether and blood is collected from the lower vein through a heparin-treated syringe. Red blood cells are isolated in conventional manner. After washing the blood cells with phosphate buffered physiological saline, they are used for experiment.

The isolated red blood cells are suspended in phosphate buffered physiological saline in a final concentration of $3.3 \times 10^7$ cells/ml and a hemolysis-inducing substance (water or lysolecithin) is added to the suspension followed by incubation at 25°C for 15 minutes. When the effects of test substances (glutathiones) are examimined, glutathiones in various concentrations and red blood

cells are pre-incubated at 25°C for 30 minutes prior to the reaction. The degree of hemolysis is determined by calculating isolated hemoglobin from red blood cells by measuring the absorbancy at 412 nm of the supernatant obtained by removing red blood cells by centrifugation at 3,000 x g after the reaction.

Results

1) Prevention of hemolysis induced when water is added:

The extent of prevention of hemolysis (induced when water is added to a phosphate buffered physiological saline of red blood cells to make 40 v/v %) by glutathione, glutathione monoethyl ester and glutathione monoisopropyl ester at various concentration is shown in Figure 1.

2) Prevention of hemolysis induced when lysolecithin is added:

The extent of prevention of hemolysis (induced when 2.6 g/ml of lysolecithin is added to a phosphate buffered physiological saline of red blood cells) by glutathione and glutathione monoisopropyl ester at various concentration is shown in Figure 2.

In Figures 1 and 2, values plotted on the vertical axis represent the isolated hemoglobin in each case as a percentage of that for the control (no addition).

Prevention of human red blood cell hemolysis

Method

Blood is collected from normal male volunteers through a syringe treated with sodium citrate and red blood cells are isolated in conventional manner. After washing with phosphate buffered physiological saline, the isolated red blood cells are provided for test.

The isolated red blood cells of each volunteer are suspended in phosphate buffered physiological saline in a final concentration of $10^8$ cells/ml. Hemolysis is induced either by adding water (46 to

50 v/v%) or lysolecithin (3 g/ml) followed by incubation at 25° C for 15 minutes or by shaking (160 times/min.) together with glass beads having a diameter of about 1 mm for an hour. When effects of test substances (glutathiones) are examined, glutathiones in various concentrations and red blood cells are preincubated at 25°C. for 30 minutes prior to the reaction. The degree of hemolysis is determined by calculating isolated hemoglobin from red blood cells by measuring the absorbancy at 412 nm of the supernatant obtained by removing red blood cells by centrifugation at 3,000 x g for 5 minutes after the reaction.

The effects of glutathiones are compared, determined by 50 % inhibitory concentration ($IC_{50}$) of hemoglobin against isolation from red blood cells based on the concentration-hemoglobin isolation prevention curve.

Results

In Table 1, the $IC_{50}$ values of glutathione, glutathione monoethyl ester and glutathione monoisopropyl ester are shown against hemolysis induced when water and lysolecithin are added and by mechanical hemolysis with glass beads.

Table 1 Protective Action against Hemolysis of Human Red Blood Cell

| Glutathiones ($IC_{50}$; mM) | Kind of Hemolysis | | |
|---|---|---|---|
| | Water (Low Tension) | Lysolecithin | Mechanical |
| Glutathione | >1 | >1 | >1 |
| Monoethyl ester | 1 | | |
| Monoisopropyl ester | 0.01 | 0.03 | 0.1 |

The invention provides a medicament comprising a pharmaceutically acceptable vehicle and at least one of the formula (I) compounds and salts. It also provides the use of formula (I) compounds and salts for the production of medicaments for the novel prevention or treatment of conditions, ailments and symptoms indicated herein, e.g. anemia. It also provides those formula (I) compounds and salts which are novel per se.

CLAIMS :

1. A pharmaceutical composition comprising a pharmaceutically acceptable vehicle and as an effective component at least one compound selected from glutathione monoalkyl esters of formula :

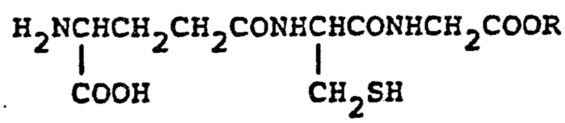

and salts thereof, wherein R represents an alkyl group.

2. A composition as claimed in claim 1 wherein R is a $C_1$ to $C_{10}$ group.

3. A composition as claimed in claim 2 wherein R is an iso-propyl group.

4. The use for the preparation of an antianemic medicament (e.g. for prevention or treatment of hemolytic anemia) of at least one compound selected from glutathione monoalkyl esters of formula

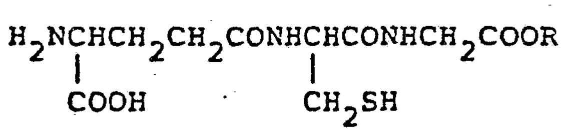

and salts thereof, wherein R represents an alkyl group.

5. A novel compound selected from glutathione monoalkyl esters of formula

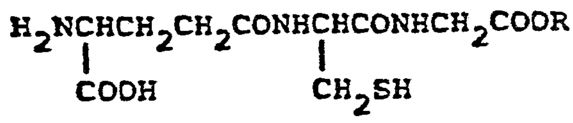

and salts thereof, wherein R represents an alkyl group.

6. A method of preparing a compound of formula

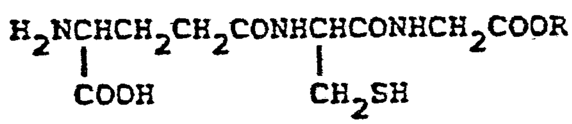

or a salt thereof, wherein R represents an alkyl group, which comprises esterifying glutathione with the corresponding alkyl alcohol and optionally subjecting the product to salt formation or desalting.

Fig. 1

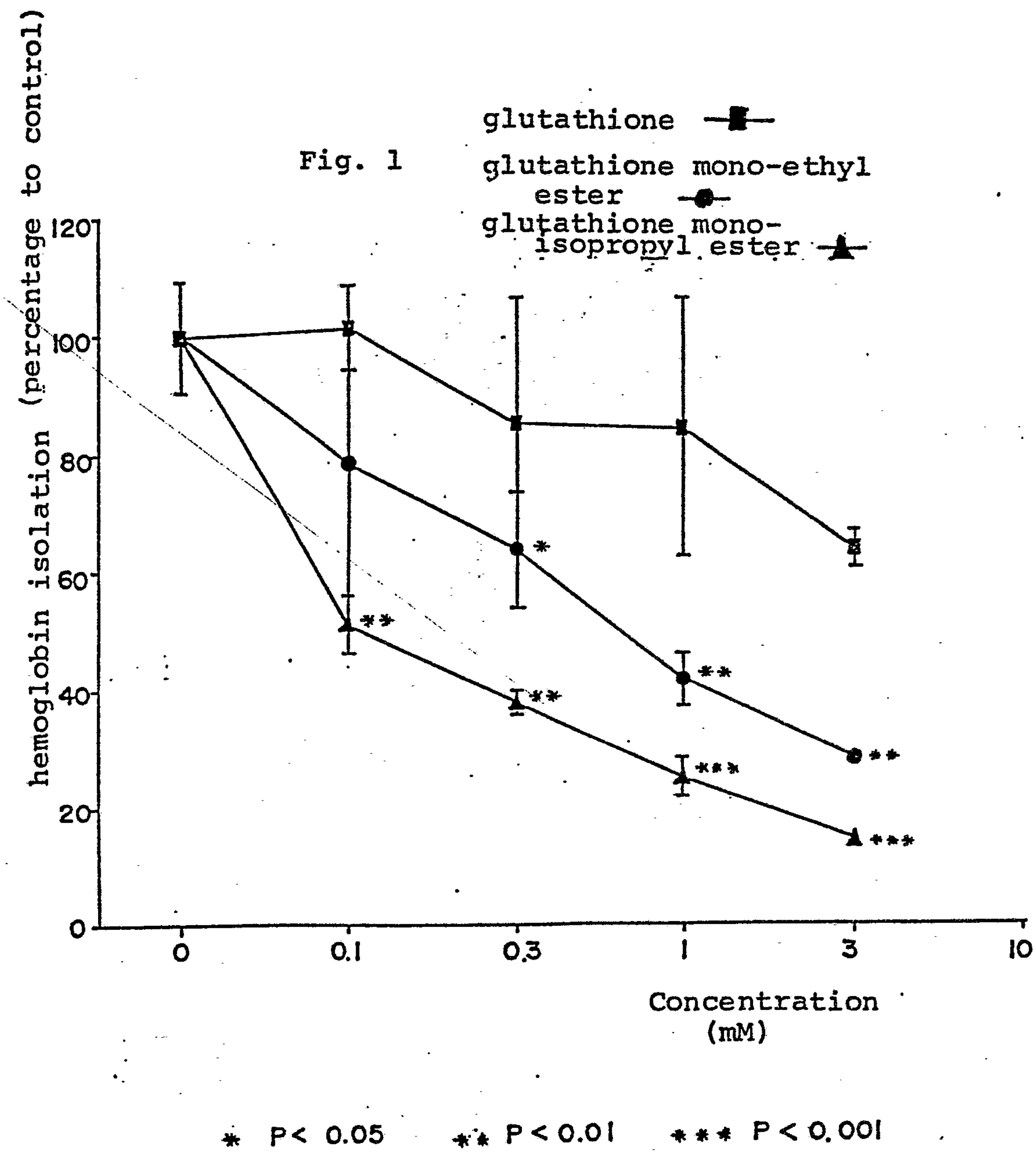
glutathione
glutathione mono-ethyl ester
glutathione mono-isopropyl ester
hemoglobin isolation (percentage to control)
120
100
80
60
40
20
0
0
0.1
0.3
1
3
10
Concentration (mM)
* P< 0.05
** P < 0.01
*** P < 0.001

Fig. 2

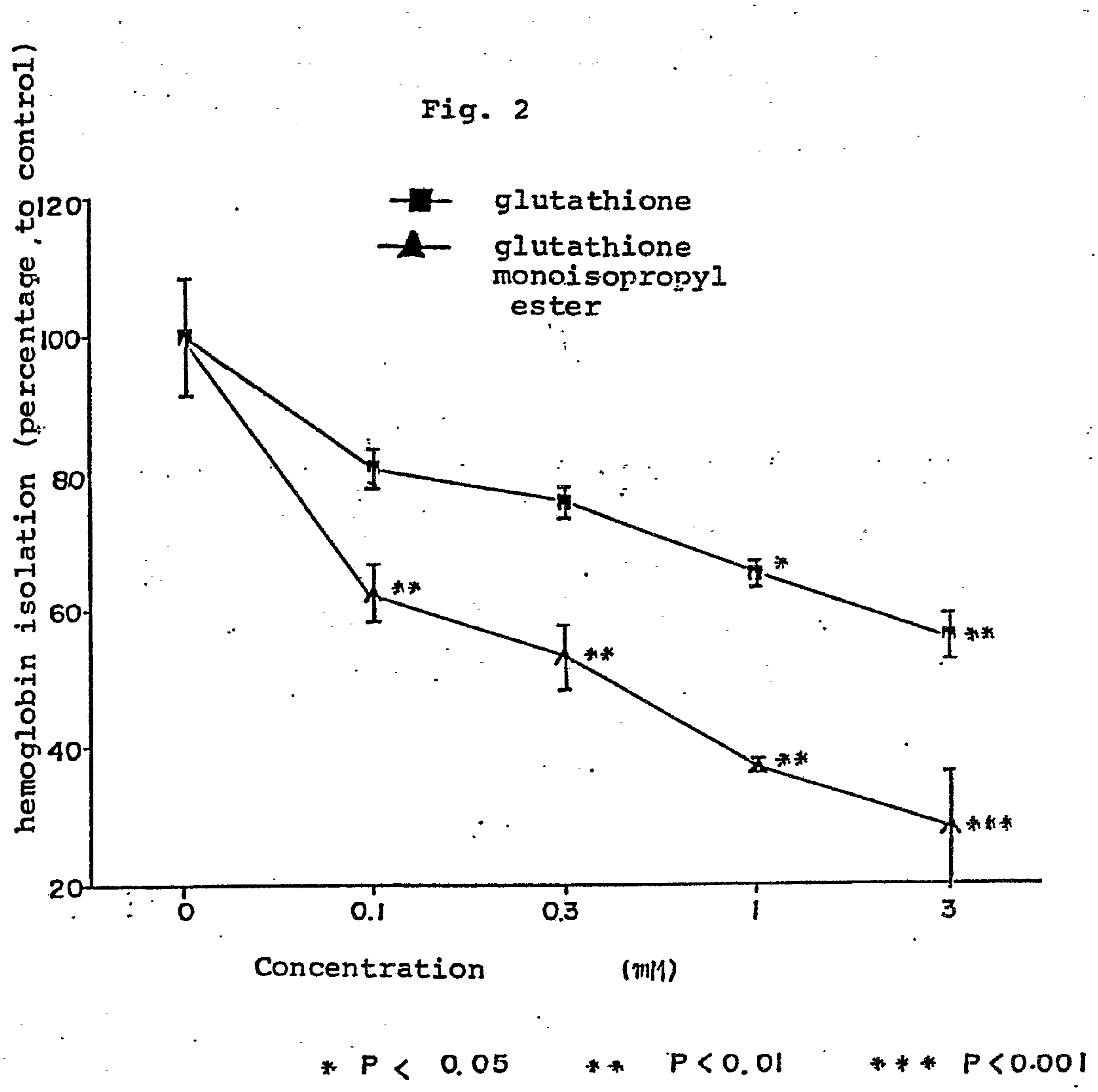
glutathione
glutathione monoisopropyl ester
hemoglobin isolation (percentage to control)
120
100
80
60
40
20
0
0.1
0.3
1
3
Concentration (mM)
* P < 0.05  ** P < 0.01  *** P < 0.001

European Patent Office

# EUROPEAN SEARCH REPORT

0249401
Application number

EP 87 30 4962

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, vol. 105, no. 17, 27th October 1986, page 743, columns 1-2, abstract no. 153558z, Columbus, Ohio, US; Y. NAGANO et al.: "Preparation of glutathione monoalkyl esters", & JP - A - 61 15870 (KOKAI TOKKYO KOHO) 23-01-1986 (Cat. X)<br>--- | 1,5 | C 07 K 5/08<br>A 61 K 31/195 |
| X | CHEMICAL ABSTRACTS, vol. 104, no. 21, 26th May 1986, page 204, column 2, abstract no. 181587f, Columbus, Ohio, US; CORNELL RESEARCH FOUNDATION, INC. "Glutathione derivatives as antidote and radioprotectant enhancer", & JP - A - 60 123 497 (KOKAI TOKKYO KOHO) 02-07-1985<br>--- | 1,5 | |
| | | | TECHNICAL FIELDS SEARCHED (Int. Cl.4) |
| X | CHEMICAL ABSTRACTS, vol. 103, no. 9, 2nd September 1985, page 319, column 1, abstract no. 67541r, Columbus, Ohio, US; M.E. ANDERSON et al.: "Glutathione monoethyl ester: preparation, uptake by tissues, and conversion to glutathione", & ARCH. BIOCHEM. BIOPHYS. 1985, 239(2), 538-48<br>----- | 1,5 | C 07 K 5/08<br>A 61 K 31/195 |

The present search report has been drawn up for all claims

| Place of search | Date of completion of the search | Examiner |
|---|---|---|
| BERLIN | 28-08-1987 | KAPTEYN H G |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82